# EUROPEAN PATENT APPLICATION

(11) **EP 1 245 228 A2**
(43) Date of publication of application: **02.10.2002**
(21) Application number: 02014620.5
(22) Date of filing: 31.01.1995
(51) Int. Cl.: A61K 31/135, A61P 25/28

(54) **Medicament for the inducement of cognition enhancement**

(30) Priority: 14.02.1994 US 195417
(62) Divisional of application: 95300612.9
(71) Applicant: Wyeth, Madison, New Jersey 07940-0874 (US)
(72) Inventor: Husbands, George Edward Morris, Berwyn, Pennsylvania 19312 (US); Abou-Gharbia, Magid Abdel-Megid, Glen Mills, Pennsylvania 19342 (US); Moyer, John Allen, New Hope, Pennsylvania 18938 (US); Muth, Eric Anthony, New Hope, Pennsylvania 18938 (US)
(74) Representative: Connelly, Michael John

(57) **Abstract**

This invention provides use of a compound to manufacture a medicament of inducing cognition enhancement. The compound is a 2-(1-hydroxycycloalkyl or 1-hydroxycycloalkenyl)-2-phenylalkylamine derivative having the formula: 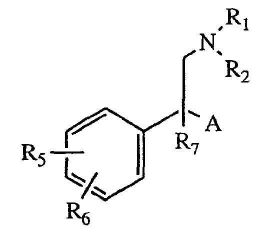 in which A is a moiety of the formula 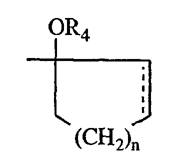 wherein
the dotted line represents optional unsaturation;
R₁ is hydrogen or alkyl;
R₂ is alkyl;
R₄ is hydrogen, alkyl, formyl, or alkanoyl;
R₅ and R₆ are, independently, hydrogen, hydroxyl, alkyl, alkoxy, alkanoyloxy, cyano, nitro, alkylmercapto, amino, alkylamino, dialkylamino, alkanamido, halo, trifluoromethyl, or taken together, methylene dioxy;
R₇ is hydrogen or alkyl; and
n is 0, 1, 2, 3, or 4;
or a pharmaceutically acceptable salt thereof.

## Description

This invention comprises a new use for venlafaxine. More particularly, this invention comprises use of venlafaxine to prepare a medicament for inducing cognition enhancement in a mammal.

The active ingredients of this invention, (1-[2-(dimethylamino)-1-(4-methoxyphenyl)ethyl]cyclohexanol), its analogues or therapeutically acceptable salts thereof, are known generally as venlafaxine. These ingredients are disclosed in U. S. Patent No. 4,535,186 (Husbands et al.) and have been previously reported to be useful as an antidepressant. U. S. Patents Nos. 4,535,186 and 5,043,466 teach the production of venlafaxine and its analogues and are incorporated herein by reference. For the purposes of this disclosure and the claims that follow, it is understood that the use of venlafaxine includes the use of venlafaxine's free base, its pharmaceutically acceptable salts, its racemate and its individual enantiomers, and venlafaxine analogs, both as racemates and as their individual enantiomers.

Venlafaxine has been shown to be a potent inhibitor of monoamine neurotransmitter uptake, a mechanism associated with clinical antidepressant activity. Due to its novel structure, venlafaxine has a mechanism of action unrelated to other available antidepressants, such as the tricyclic antidepressants desipramine, nortriptyline, protriptyline, imipramine, amitryptyline, trimipramine, and doxepin.

It is believed that venlafaxine's mechanism of action is related to potent inhibition of the uptake of the monoamine neurotransmitters serotonin and norepinephrine. To a lesser degree, venlafaxine also inhibits dopamine reuptake, but it has no inhibitory activity on monoamine oxidase. O-desmethylvenlafaxine, venlafaxine's major metabolite in humans, exhibits a similar pharmacologic profile. Venlafaxine's ability to inhibit norepinephrine and serotonin (5-HT) uptake has been predicted to have an efficacy which rivals or surpasses that of tricyclic antidepressants (Stuart A. Montgomery, M.D., J. Clin. Psychiatry, 54:3, March 1993).

In contrast to classical tricyclic antidepressant drugs, venlafaxine has virtually no affinity for muscarinic, histaminergic, or adrenergic receptors in vitro. Pharmacologic activity at these receptors is associated with the various anticholinergic, sedative and cardiovascular effects seen with the tricyclic antidepressant drugs.

The present invention provides use of a compound to manufacture a medicament for inducing cognition enhancement in a mammal, preferably in a human. This invention may also be referred to as use to manufacture a medicament for treating cognitive impairment in a mammal, preferably in a human, such as, but not limited to the cognitive impairments caused by dementias, Alzheimer's-type Dementia, Parkinson's Disease and Age Associated Memory Impairment. Symptoms of such cognition-depleting maladies may include varying reductions in concentration, judgement, memory and orientation.

In accordance with the present invention there is provided use of a substituted phenethylamine to manufacture a medicament for inducing cognition enhancement. The enhancement may be in a mammal, preferably in a human. The compound used is a compound having the formula: 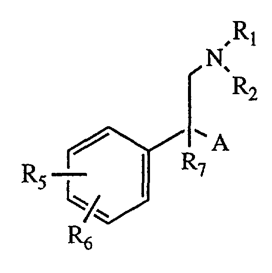 in which A is a moity of the formula 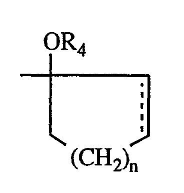 wherein
the dotted line represents optional unsaturation;
R₁ is hydrogen or alkyl of 1 to 6 carbon atoms;
R₂ is alkyl of 1 to 6 carbon atoms;
R₄ is hydrogen, alkyl of 1 to 6 carbon atoms, formyl, or alkanoyl of 2 to 7 carbon atoms;
R₅ and R₆ are independently hydrogen, hydroxyl, alkyl of 1 to 6 carbon atoms, alkoxy of 1 to 6 carbon atoms, alkanoyloxy of 2 to 7 carbon atoms, cyano, nitro, alkylmercapto of 1 to 6 carbon atoms, amino, alkyl-amino of 1 to 6 carbon atoms, dialkylamino in which each alkyl group is of 1 to 6 carbon atoms, alkanamido of 2 to 7 carbon atoms, halo, trifluoromethyl, or when taken together, methylene dioxy;
R₇ is hydrogen or alkyl of 1 to 6 carbon atoms; and n is 0, 1, 2, 3, or 4; or a pharmaceutically acceptable salt thereof.

The preferred compounds are those of the formula: 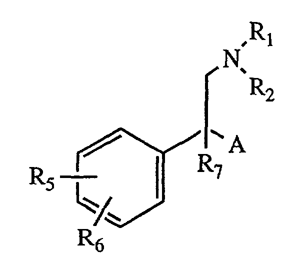 in which
A is as defined supra;
R₁ is hydrogen or alkyl of 1 to 3 carbon atoms;
R₂ is alkyl of 1 to 3 carbon atoms;
R₅ is hydrogen, hydroxyl, alkoxy of 1 to 3 carbon atoms, chloro, bromo, trifluoromethyl or alkyl of 1 to 3 carbon atoms;
R₆ is alkyl of 1 to 3 carbon atoms, alkoxy of 1 to 3 carbon atoms, chloro, bromo, trifluoromethyl or alkanoyloxy of 2 to 3 carbon atoms;
R₇ is hydrogen or alkyl of 1 to 3 carbon atoms; or a pharmaceutically acceptable salt thereof.

The most preferred compounds are those in which both R₅ and R₆ are in meta positions, or one of R₅ and R₆ is in the para position, and n is 2.

Of particular interest are the compounds 1-[(2-dimethylamino)-1-(4-methoxyphenyl)ethyl]cyclohexanol and 1-[(2-dimethylamino)-1-(4-hydroxyphenyl)-ethyl]cyclohexanol and pharmaceutically acceptable salts thereof.

The compounds in which R₄ is formyl or alkanoyl of 2 to 7 carbon atoms have been found to be not as potent as the corresponding free hydroxy bearing derivatives. However, in long term therapy the acyloxy derivatives will act as pro drugs as the acyl group is removed in vivo either via acid hydrolysis in the stomach or enzymatically.

The pharmaceutically acceptable acid addition salts of the basic compounds of this invention are formed conventionally by reaction of the free base with an equivalent amount of any acid which forms a non-toxic salt. Illustrative acids are either inorganic or organic, including hydrochloric, hydrobromic, fumaric, maleic, succinic, sulfuric, phosphoric, tartaric, acetic, citric, oxalic, and similar acids. For parenteral administration, the use of water soluble salts is preferred, although either the free base of the pharmaceutically acceptable salts are applicable for oral or parenteral administration of the cognition enhancing agents of this invention. The halo substituent representing R₅ or R₆ is intended to include the chloro, bromo, iodo, or fluoro substituents.

Pharmaceutical compositions can be prepared with the compounds of this invention as active ingredients. The active ingredient can be compounded into any of the usual oral dosage forms including tablets, capsules and liquid preparations such as elixirs and suspensions containing various coloring, flavoring, stabilizing and flavor masking substances. For compounding oral dosage forms, the active ingredient can be mixed with various conventional tabletting materials such as starch, calcium carbonate, lactose, sucrose and dicalcium phosphate to aid the tabletting or capsulating process. Magnesium stearate, as an additive, provides a useful lubricant function when desired.

The active ingredients can be dissolved or suspended in a pharmaceutically acceptable sterile liquid carrier, such as sterile water, sterile organic solvent or a mixture of both. Preferably a liquid carrier is one suitable for parenteral injection. Where the active ingredient is sufficiently soluble it can be dissolved in normal saline as a carrier; if it is too insoluble for this it can often be dissolved in a suitable organic solvent, for instance aqueous propylene glycol or polyethylene glycol solutions. Aqueous propylene glycol containing from 10 to 75% of the glycol by weight is generally suitable. In other instances other compositions can be made by dispersing the finely-divided active ingredient in aqueous starch or sodium carboxymethyl cellulose solution, or in a suitable oil, for instance arachis oil. Liquid pharmaceutical compositions which are sterile solutions or suspensions can be utilized by intramuscular, intraperitoneal or subcutaneous injection.

Preferably the pharmaceutical composition is in unit dosage form, e.g. as tablets or capsules. In such form, the composition is sub-divided in unit doses containing appropriate quantities of the active ingredient; the unit dosage forms can be packaged compositions, for example, packeted powders or vials or ampoules. The unit dosage form can be a capsule, cachet or tablet itself, or it can be the appropriate number of any of these in package form. The quantity of the active ingredient in a unit dose of composition may be varied or adjusted from about 2 mg. or less to about 50 mg. or more, according to the particular need and the activity of the active ingredient. The usual oral recommended dose of venlafaxine for humans may be between about 75 and about 200 mg/day and this dose may be administered in divided doses, preferably with food if administered orally. A maximum recommended daily dose for humans would be about 375 mg. It will be understood by one skilled in the art that dosage under this invention will be determined by the particular circumstances surrounding each case, as will the route of administration.

It should also be understood that the present invention is intended to include all methods of, and reasons for, inducing cognition enhancement in a mammal by administering to the mammal an effective amount of venlafaxine or its analogues or pharmaceutically acceptable salts. For the purposes of the present invention, inducing cognition enhancement is to be understood as covering all prophylactic, therapeutic, progression inhibiting, remedial, maintenance, curative or other administrations, regimens or treatments of or with venlafaxine or its analogues or salts that yield the desired cognition enhancing effects in a mammal.

The following example is provided to demonstrate the efficacy of venlafaxine in the production of cognitive enhancing qualities in a mammal. It is understood that this example is merely illustrative and is not intended to limit the scope of the present invention.

### Cognition Enhancement Test

To establish venlafaxine's cognition enhancement properties, it was tested in the scopolamine-impaired radial arm maze test. In this test, a reduction in scopolamine impairment of memory is indicative of cognitive enhancement.

### Materials and Methods

### Animals

Male Sprague-Dawley, CD rats (Charles River, Kingston, NY) weighing 200-250 g on arrival were used. For one week, the rats were housed, six per cage, with standard laboratory chow and water available ad libitum. Housing was in a colony room maintained at 22°C and had a 12 hour light/dark cycle with lights on at 6:00 AM. Following habituation to the facility, animals were individually housed and maintained at 85% of free-feeding weight (Results® precision pellets, Bio-Serv, Frenchtown, NJ). Once stable weights were attained, the rats were acclimated to the 8-arm radial maze.

### Materials

The structure of the maze was an adaptation from that of Peele and Baron (Pharmacology, Biochemistry, and Behavior, 29:143-150, 1988). The maze was elevated to a height of 75.5 cm and composed of a circular area surrounded by 8 arms radiating away from the center, equidistant from one another. Each arm was 58 cm long x 13 cm high. A clear plexiglass cylinder was lowered to enclose the animal in the center portion of the maze prior to the start of each session. Each arm of the maze was equipped with 3 sets of photocells interfaced to a data acquisition unit (Hewlett Packard 2497A), which in turn was interfaced to an HP Vectra ES/12. The photocells were used to track the movement of the rat in the maze. Coulburn Pellet Feeders (E14-12) located above food cups at the end of each arm, dispensed two 45 mg chocolate pellets (Bio-Serv) when the outer photocell of the arm was activated for the first time in a given session. An in-house program compiled and stored the data. The maze was located in a testing room with black and white geometric posters on each wall to serve as visual cues. During all training and testing procedures, white noise was audible (∼ 70 db).

### Procedure

The training procedure consisted of five phases, each with daily sessions lasting 5 or 10 minutes. A 10 second delay was imposed between the time the rat was placed in the center portion of the maze and when the cylinder was raised to begin the session. During Phase 1, food-restricted pairs of rats were placed on the maze for 10 minutes with 45 mg chocolate food pellets scattered throughout the 8 arms of the maze. During Phase II, each rat was placed individually on the maze for a 10 minute period, with pellets scattered from the middle photocell to the food cup of each arm. During Phase III, each rat was placed on the maze for a 10 minute period, with food pellets located only in and around the food cups in each arm. In Phase IV, each rat was allowed 10 minutes to collect two pellets from each arm. Re-entry into an arm was considered an error. Rats were trained daily in this manner until they achieved criterion performance with ≤ 2 total errors on three consecutive days of training. Total habituation and training time was approximately 3 weeks.

### Drug Preparation

Venlafaxine was used in the form of the hydrochloride salt of 1-[2-(dimethylamino)-1-(4-methoxyphenyl)ethyl]cyclohexanol. Venlafaxine was prepared in phosphate buffered saline and administered in a volume of 1 ml/kg. Scopolamine HBr (0.3 mg/kg s.c.) served as the impairing agent, producing an increase in error rate (loss of memory). In preliminary experiments it was determined that 10 mg/kg i.p. of venlafaxine was the highest dose of the drug which, in combination with scopolamine (0.3 mg/kg s.c.), was tolerated by the rats, as evidenced by appropriate completion of the test. In the experiments reported here, venlafaxine (1, 3, 10 mg/kg i.p.) was given intraperitoneally simultaneously with scopolamine, 30 minutes prior to the first maze exposure on any given test day.

### Statistical Design

To assess venlafaxine, an 8 x 8 balanced latin square for repeated measures was designed, in order to achieve a high experimental efficiency with the least amount of animals. Eight experimental sessions, two per week, were conducted with the 8 treatments (vehicle, scopolamine, 3 doses of venlafaxine in combination with scopolamine) randomized within each session. Each treatment followed every other treatment the same number of times. Therefore, the residual effect of every treatment could be estimated and removed from the direct treatment effect. Following ANOVA, multiple comparisons were performed using Dunnett's two-sided test on adjusted means.

Animals that did not make 4 correct choices within 5 minutes during the first exposure, or that had not made a total of 8 choices by the end of the 2nd exposure, were considered to have "timed-out" for that session. Any animal that "timed-out" following administration of more than one dose of venlafaxine was excluded from the analysis.

### Results

Venlafaxine (3.0 mg/kg i.p.) produced significant reductions in scopolamine impairment. The 10 mg/kg i.p. dose of venlafaxine also attenuated the scopolamine impairment, since this group did not differ significantly from vehicle-treated (i.e., no scopolamine) animals.

In order to estimate ED₅₀ values for the effects of venlafaxine on the scopolamine impairment, dose-response curves were fit to the mean error data. To fit these curves, the effect of scopolamine by itself was used as an indication of the maximum impairment (upper asymptote of curves). Since there was no drug effect on this condition, the drug effect was considered to be 0%. The 3 mg/kg dose of venlafaxine produced the largest degree of reduction in the scopolamine impairment (lower asymptote of curves) and was used as an indication of the maximum (i.e., 100%) drug effect. ED₅₀ values were then estimated graphically to be 1 mg/kg i.p. for venlafaxine. As a comparison, idebenone was determined to have an ED₅₀ value of 3 mg/kg i.p.

## Claims

1. Use of a compound to manufacture a medicament for inducing cognition enhancement, wherein the said compound is a compound having the formula: 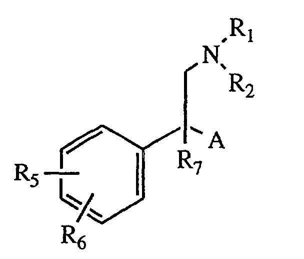 in which A is a moiety of the formula 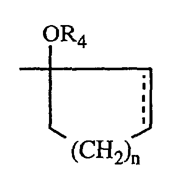 wherein
the dotted line represents optional unsaturation;
R₁ is hydrogen or alkyl of 1 to 6 carbon atoms;
R₂ is alkyl of 1 to 6 carbon atoms;
R₄ is hydrogen, alkyl of 1 to 6 carbon atoms, formyl, or alkanoyl of 2 to 7 carbon atoms;
R₅ and R₆ are, independently, hydrogen, hydroxyl, alkyl of 1 to 6 carbon atoms, alkoxy of 1 to 6 carbon atoms, alkanoyloxy of 2 to 7 carbon atoms, cyano, nitro, alkylmercapto of 1 to 6 carbon atoms, amino, alkylamino of 1 to 6 carbon atoms, dialkylamino in which each alkyl group is of 1 to 6 carbon atoms, alkanamido of 2 to 7 carbon atoms, halo, trifluoromethyl, or taken together, methylene dioxy;
R₇ is hydrogen or alkyl of 1 to 6 carbon atoms; and
n is 0, 1, 2, 3, or 4;
or a pharmaceutically acceptable salt thereof.

2. Use as claimed in Claim 1 in which the medicament is for inducing cognition enhancement in a human.

3. Use as claimed in Claim 1 wherein the compound is a compound having the formula: 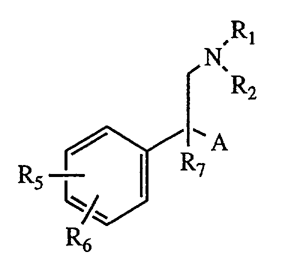 in which A is as defined in Claim 1;
R₁ is hydrogen or alkyl of 1 to 3 carbon atoms;
R₂ is alkyl of 1 to 3 carbon atoms;
R₅ is hydrogen, hydroxyl, alkoxy of 1 to 3 carbon atoms, chloro, bromo, trifluoromethyl or alkyl of 1 to 3 carbon atoms;
R₆ is alkyl of 1 to 3 carbon atoms, alkoxy of 1 to 3 carbon atoms, chloro, bromo, trifluoromethyl or alkanoyloxy of 2 to 3 carbon atoms; and
R₇ is hydrogen or alkyl of 1 to 3 carbon atoms;
or a pharmaceutically acceptable salt thereof.

4. Use as claimed in Claim 3 wherein R₅ and R₆ are both in meta positions, or one of R₅ and R₆ is in the para position, and n is 2.

5. Use as claimed in Claim 3 wherein the compound is 1-[(2-dimethylamino)-1-(4-methoxyphenyl)ethyl]cyclohexanol or a pharmaceutically acceptable salt thereof.

6. Use as claimed in Claim 3 wherein the compound is 1-[2-(dimethylamino)-1-(4-hydroxyphenyl)ethyl]cyclohexanol or a pharmaceutically acceptable salt thereof.

7. Use as claimed in any one of Claims 3 to 6, wherein the medicament is for inducing cognition enhancement in a human.

8. Use as claimed in any one of Claims 1 to 7, wherein the said compound is to be administered at a dose of between about 50 mg/day and about 375 mg/day.

9. Use as claimed in Claim 8, wherein the said compound is to be administered at a dose of between about 75 mg/day and about 200 mg/day.
